# EUROPEAN PATENT SPECIFICATION

(11) **EP 3 946 124 B1**
(45) Date of publication and mention of the grant of the patent: **16.10.2024**
(21) Application number: 20719556.1
(22) Date of filing: 31.03.2020
(51) Int. Cl.: A61B 18/18

(54) **MICROWAVE TREATMENT SYSTEM**
MIKROWELLENBEHANDLUNGSSYSTEM
SYSTÈME DE TRAITEMENT À MICRO-ONDES

(30) Priority: 02.04.2019 GB 201904645
(43) Date of publication of application: 09.02.2022
(73) Proprietor: Emblation Limited, Inglewood, Alloa FK10 2HU (GB)
(72) Inventor: MCERLEAN, Eamon, Alloa FK10 (GB); KIDD, Matthew, Alloa FK10 (GB); BEALE, Gary, Alloa FK10 (GB); JOSHI, Shailesh, Alloa FK10 (GB)
(74) Representative: Marks & Clerk LLP
(86) International application number: PCT/EP2020/059074
(87) International publication number: WO 2020/201261

(56) References cited:
- GB-A- 2 441 306
- US-A1- 2008 300 588
- US-A1- 2009 171 424
- US-A1- 2012 071 794
- US-A1- 2012 277 763
- US-A1- 2013 223 702

## Description

### Field

The present invention relates to a microwave system, for example, a microwave treatment system.

### Background

In most energy-based treatment systems, such as electromagnetic ablation systems using microwaves, radiation is delivered from a radiation generator, via a connecting cable, to a radiation delivering applicator placed in or on a tissue.

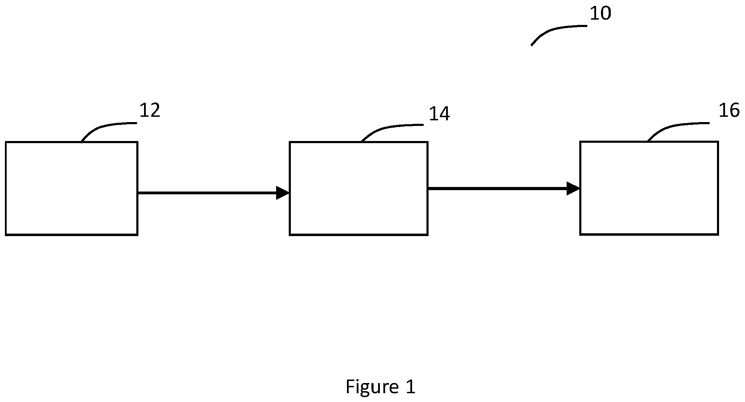

A microwave radiation delivery system 10 for treating biological tissue is shown in Fig 1. The system has a microwave generator 12 for generating microwave radiation, a flexible interconnecting cable 14 and a microwave applicator 16.

Typically, the applicator 16 is inserted deep within the tissue when treating internal organs, for example hepatic tumour ablation. When treating the tissue on the surface such as skin tissue for a dermatological condition, the applicator is placed on the tissue externally and radiation is delivered to the surface.

Dermatological conditions such as small warts and papilloma exhibit an area of abnormal growth that is relatively small in size, for example a diameter of less than 10mm or an active area of 100 mm² or less. In the following, the term lesion is used to refer collectively to a surface area of biological tissue affected with an adverse biological condition. Treating these conditions with radiation devices is achievable with an applicator possessing a radiating or effective area identical in size or slightly bigger than the lesions. This helps deliver the radiation to the entire surface of the lesion in a single session.

Similarly, multiple smaller lesions can be treated individually by applying radiation with a variable magnitude usually depending upon the severity and requirement for treatment of the condition. However, the distribution of energy density may possibly be uneven on the surface of the lesion resulting in under and over treatment. It may also result in treating healthy tissue surrounding the lesion.

On the other hand, many biological conditions such as psoriasis, eczema, dermatitis are relatively larger in size and comprise a large surface area that requires treatment. To target these lesions with an applicator that is smaller in size than the lesion requires multiple treatments of the affected area to ensure sufficient coverage. A significant drawback of this approach may be over-treating certain areas resulting in undesirable events such as damage to the tissue, for example, this may lead to hematoma, blistering. Another drawback to multiple single treatments may be under-treatment of a certain area leaving behind the affected condition partially untreated. Performing multiple single treatments may also be time-consuming.

As an alternative, a smaller applicator may be moved continuously on the lesion surface with a simultaneous radiation of energy. This may be more efficient however, this may lead to an uneven treatment of the lesion. An uneven energy density in the region being treated may compromise the efficacy of the treatment.

One way to address the issue of using small applicators to treat a large lesion may be to utilise applicators of a larger size so as to cover the entire region of the affected area. However, considering the variations in the sizes of these biological conditions within a patient and across a spectrum of patients, it may be impractical to design and manufacture such bespoke applicators based upon the size and shape of a range of lesions. US 2009/171424A1 describes an applicator which mechanically vibrates when RF power is applied. US 2012/277763A1 describes a dynamic ablation device. GB 2441306A describes a movable microwaveable applicator. US 2008/300588 describes using an ablation path. US 2013/223702A1 describes a surgical instrument navigation system.

### Summary

The invention and its most advantageous embodiments are defined in the appended set of claims.

In accordance with a first aspect of the invention, there is provided a microwave system, comprising:
a microwave generator;
a microwave applicator for delivering microwave radiation generated by the microwave generator to a surface, wherein the microwave applicator is moveable relative to the surface;
one or more sensors for sensing at least one of a position, an orientation, an acceleration, a speed and/or a velocity of the microwave applicator, and
a controller configured to monitor sensor output from the one or more sensors and further configured to control one or more operational parameters of the microwave system based at least on the monitored sensor output.

The controller is configured to control one or more operational parameters of the microwave generator when the applicator is at a position based on the time elapsed since the applicator was previously at said position.

The controller may be configured to monitor sensor output from the one or more sensors, for example, as the applicator is moved relative to the surface.

The one or more sensors may be further configured for sensing at least one of a position, an orientation, an acceleration, a speed and/or a velocity of the surface.

The controller may be configured to control the one or more operational parameters thereby to maintain, increase and/or decrease a power or energy of the generated microwave radiation in response to the applicator being moved relative to the surface. The controller may be configured to deliver microwave radiation to the surface in accordance with a pre-determined dosage.

The controller may be configured to control the one or more operational parameters thereby to maintain, increase and/or decrease a power or energy of the generated microwave radiation in response to the applicator being moved relative to the surface thereby to provider microwave radiation to the surface in accordance with a pre-determined dose. The dose may comprise, for example, a distribution of energy in space, or a distribution of energy in time.

The controller may be configured to change the one or more operational parameters in response to a change in one or more of the position, orientation, velocity, or acceleration of the applicator.

The controller may be configured to control the one or more operational parameters of the microwave generator such that the microwave radiation delivered across the surface comprises a substantially constant energy density.

The controller is configured to control one or more operational parameters of the microwave generator when the applicator is at a position based on the time elapsed since the applicator was previously at said position.

The controller may be configured to control the one or more operational parameters of the microwave generator based on the sensor output so that the delivered microwave radiation maintains a temperature of the surface within a pre-determined temperature window.

The controller may be further configured to cut-off the microwave radiation or at least reduce the amount of microwave radiation that is generated by the microwave generator in response to at least one of:
a) the sensor output being representative of the microwave applicator being outside a pre-determined treatment region;
b) the sensor output being representative of the microwave applicator being at a height above a surface that is above a pre-determined threshold;
c) the sensor output being representative of the microwave applicator being at a position, or moving at a velocity or having an acceleration above an upper threshold and/or below a lower threshold;
d) the sensor output being representative of the microwave applicator being oriented away from a desired axis relative to the surface.

The microwave applicator may be coupled to the microwave generator by a cable assembly.

The generated microwave radiation may comprise a series of pulsed microwave signals and the controller is configured to control the generation of microwave radiation by varying one or more pulse parameters. The one or more pulse parameters may comprise at least one of: a pulse width, a pulse frequency, a pulse height, a pulse duration.

The generated microwave radiation may comprise modulated microwave signals and the controller is configured to control the generation of microwave radiation by varying one or more modulation parameters.

The modulated microwave signals may be modulated in accordance with a modulation scheme to vary the average power delivered. The modulation scheme may comprise at least one of: amplitude modulation pulsing, pulse-width modulation and/or on off keying. The output may be modulated by control of linear gain to create a variable amplitude control of power.

The microwave applicator may be moveable by a user.

The controller may be further configured to control the microwave generator so that that the applicator delivers microwave radiation to a pre-determined treatment region of the tissue surface. The controller may be configured to reduce the microwave radiation generated when the applicator is outside the treatment region. The treatment region may be in two-dimensions or three dimensions. The treatment region may be a treatment area or a treatment volume. The treatment region may be a two-dimensional surface in three-dimensional space. The treatment area may have a height parameter.

The system may further comprise mapping circuitry configured to receive sensor output obtained during a mapping process and to determine said treatment region based at least on said sensor output. The treatment region may be a three dimensional volume. Determining the treatment region may comprise projecting three dimensional coordinates to a two dimensional surface. The treatment region may be represented by two-dimensional or three dimensional co-ordinates. The treatment region may be represented by one or more of height, width and/or length of the treatment region.

More than one treatment region may be determined. The generator may be controlled to generate microwave radiation comprising surface radiation densities for each respective treatment region.

The mapping process may comprise moving the applicator to trace a boundary of the treatment area in a two or three dimensional coordinate system.

The microwave generator may be configured to delivery microwave radiation comprising a frequency between 900 MHz and 30 GHz, optionally wherein the frequency is about 915 MHz, about 2.45 GHz, about 5.8 GHz, about 8.0 GHz, or about 24.125 GHz.

The generated microwave power could range from 0.1W to 100W but preferentially between 1W to 20W. The treatment time may range from one thousandth of a second to 1s and 1s to 1800s but preferably between 1s-10s. Surface power density may range from 1mW/mm² to 20W/mm². Surface energy density may range from 1mJ/mm² to 200J/mm².

The microwave generator may be configured to deliver one of microwave radiation at a fixed frequency, microwave radiation at a variable frequency and/or modulated microwave radiation.

The microwave applicator may comprise a radiating antenna.

The system may further comprise an indicator for providing an indication in response to at least one of the one or more sensor outputs being above and/or below a threshold.

At least one of the controller and sensors may be provided as part of the applicator.

Wireless communication means may be provided for wirelessly communicating sensor data between the applicator and the controller.

The one or more sensors may be configured to sense data in 6 degrees of freedom.

The one or more sensors may comprise at least one of:
inertial measurement unit (IMU) sensors, Microelectromechanical systems (MEMS), accelerometer sensor, gyroscope sensor, an optical tracking system, an electromagnetic tracking system, a time of flight system (ToF), a Light Detection and Ranging system (Lidar system), a Doppler radar system.

The system may comprise marker detecting means for detecting the presence and/or position of one or more active or passive markers.

The system may further comprise a display for displaying a 2D or 3D map of the treatment area. The display may be updated based on sensor output to display treated and/or untreated areas. The map may be one of two dimensional, three dimensional or four dimensional.

In accordance with a second aspect of the invention, there is provided a method of controlling a microwave system comprising a microwave applicator configured to deliver microwave radiation generated by a microwave generator to a surface, wherein the microwave applicator is further configured to be moved relative to the surface, wherein the method further comprises:
sensing at least one of a position, an orientation, an acceleration, a speed and/or a velocity of the microwave applicator;
monitoring sensor output from the one or more sensors;
controlling one or more operational parameters of the microwave system based at least on the monitored sensor output.

In accordance with a third aspect of the invention, there is provided a non-transitory computer readable medium comprising instructions operable by a processor to perform a method of controlling a microwave system, wherein the microwave system comprises a microwave applicator configured to deliver microwave radiation generated by a microwave generator to a surface and the microwave applicator is further configured to be moved relative to the surface, wherein the method comprises:
monitoring sensor output from one or more sensors, wherein the sensor output from the one or more sensors is representative of at least one of a position, an orientation, an acceleration, a speed and/or a velocity of a microwave applicator;
controlling one or more operational parameters of a microwave system based at least on the monitored sensor output.

Features in one aspect may be applied as features in any other aspect, in any appropriate combination. For example, system features may be provided as method features or vice versa.

### BRIEF DESCRIPTION OF THE DRAWINGS

Embodiments of the invention are described in relation to the following figures, in which:
Figure 1 is a schematic illustration of a microwave radiation delivery system;
Figure 2 is a schematic illustration of a microwave treatment system, in accordance with embodiments;
Figure 3 is an illustrative flowchart showing a surface mapping process and a treatment process using the microwave treatment system;
Figure 4 is a cross-sectional view of a microwave applicator assembly forming part of the microwave treatment system, in accordance with embodiments;
Figure 5 is a cross-sectional view of the microwave applicator assembly;
Figure 6 illustrates a mapping process performed by the microwave applicator assembly;
Figure 7 illustrates a mapping process performed by the microwave applicator assembly;
Figure 8 illustrates a treatment of tissue using the microwave treatment system, in accordance with embodiments;
Figure 9 shows pulse width modulation, and
Figure 10 illustrates conditions for ceasing the microwave power being radiated into the tissue being treated, and
Figures 11 to 15 illustrates a cross sectional view of an optical tracking system with an external camera system and trackers, in accordance with further embodiments.

### DETAILED DESCRIPTION OF THE DRAWINGS

A method and system for treating biological tissue with a controlled energy density using one or more surface applicator types and a modulated power source is hereby described. A large or larger surface region of lesion hereon refers to the surface area of lesion larger than the surface area of an applicator. In the following, the term lesion is used to refer collectively to a surface area of biological tissue affected with an adverse biological condition.

In overview, a system of delivering controlled energy density using a single applicator over a large surface area of tissue is described. The system entails the use of sensors for obtaining position, acceleration and velocity of the applicator, for example, IMU (inertial measurement unit) and/or MEMS (Microelectromechanical systems) accelerometers and gyroscope. The microwave radiation applicator is moved along the surface of tissue continuously and the apparatus uses the motion of the applicator dataset to modify one or more operational parameters of the microwave generator, for example, to increase or decrease the amount of applied power in real time in a closed-loop system thereby to control the generated microwave radiation delivered to the region being treated.

By controlling the source of the microwave radiation, the microwave radiation delivered by the applicator to tissue is controlled and can be delivered in accordance with a pre-determined dose. The dose may include a spatial distribution of radiation or a temporal distribution of radiation. In accordance with embodiments, the energy dose is characterised by the energy density of the radiation delivered by the applicator and this may be, for example, uniform, non-uniform, optimum, or pre-set microwave energy density. The radiation is applied into tissues and/or onto tissue surface for medical applications. Uniform, non-uniform and optimum energy density may also be collectively referred to as a controlled energy density.

By controlling the applied microwave radiation, the over-treatment and/or under-treatment of an affected area may be reduced.

In the following, energy density may refer to the amount of energy in a given volume or area. The energy density can be, for example, a two dimensional or a three-dimensional energy density. The energy density may be have units, for example, of Joules/millimeter² (J/mm²) and or Joules/millimeter³ (J/mm³).

In the following, microwave treatment refers collectively to applying microwave radiation, for example, for inducing therapeutically effective hyperthermia, ablation, sub-ablation, non-ablation, coagulation and non-coagulation in biological tissues.

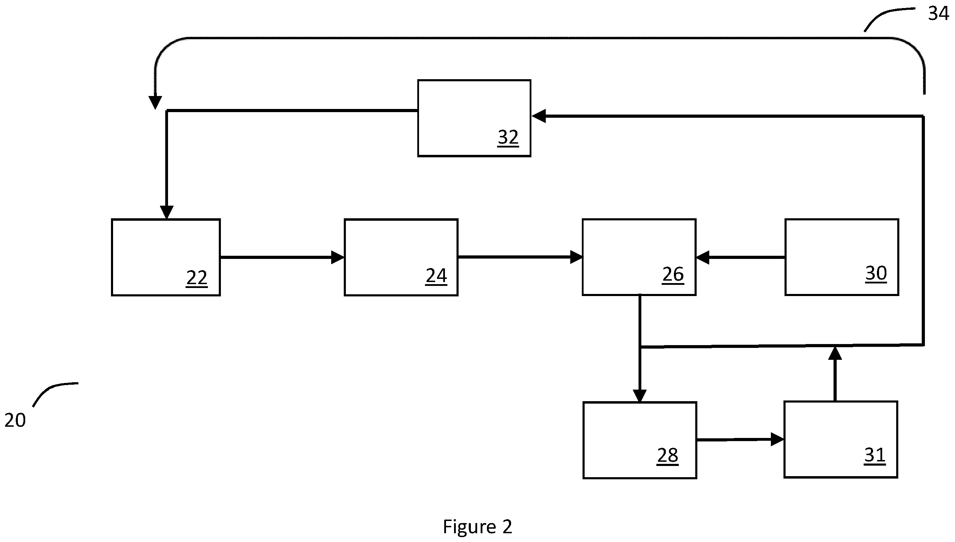

A microwave radiation delivery system 20, in accordance with embodiments, using closed loop feedback for treating a biological tissue, is illustrated in Figure 2. The system comprises of microwave generator 22 for generating microwave radiation, a flexible or rigid interconnecting cable 24 and microwave applicator assembly 26 for delivering microwave radiation to a biological tissue 28. Microwave applicator assembly 26 is equipped with a sensor unit 30 which records at least one of position, acceleration, velocity and orientation of the said microwave applicator 26. The microwave applicator assembly 26 may also be simply referred to as a microwave applicator. In the present embodiment, the sensor unit is an inertial measurement unit (IMU) and may be referred to as IMU sensor unit 30.

Additionally, in the present embodiment, biological tissue 28 is equipped with a further sensor unit 31 which records at least one of position, acceleration, velocity and orientation of the biological tissue 28.

A control system unit 32, which may also be referred to simply as a controller, is also provided as part of the system 20. The controller 32 is in communication with the sensor unit 30, further sensor unit 31 and the microwave generator 22. In the present embodiment, the communication is via wired connections. It will be understood that any suitable connection may be used. The controller 32 has monitoring circuitry for monitoring sensor output.

The control system unit 32 forms part of a feedback driven closed loop, indicated by 34. The feedback driven closed loop 34 monitors and executes controlled radiation delivery from the microwave applicator 26 to the biological tissue 28 being treated.

Depending on the tissue and/or the condition that is being treated the radiation may be delivered on to a surface or into a surface.

The controller 32 has processing circuitry or other suitable processing resource for monitoring sensor output from sensor unit 30 and the further sensor unit 31. The processing circuity is configured to process the sensor output and provide control signals to the microwave generator 22 based on the monitored sensor output.

In the present embodiment, the processing circuitry is configured to process sensor output from the sensor unit 30 and from the further sensor unit 31 and provide control signals to the microwave generator 22 based on the monitored sensor output.

In other embodiments the processing circuitry is configured to process sensor output from the sensor unit 30 and provide control signals to the microwave generator 22 based on the monitored sensor output from the sensor unit 30.

The microwave generator 22 provides a source of microwave radiation that is provided to the applicator 26 via the cable 24. The control signals from the controller 32 are representative of instructions to change the operational parameters of the microwave generator 22. The microwave generator 22 is controlled by changing or selecting one or more operational parameters.

In some embodiments, the microwave generator 22 produces microwave radiation using a series of pulsed signals. The controller 32 can control the generation of microwave radiation by varying one or more properties of the pulsed signals, for example, pulse duration, pulse width, pulse frequency, pulse period.

Applied radiation may be in the form of a continuous oscillating electromagnetic wave (CW) at a fixed frequency or a modulated (variable frequency). Pulse regimes include amplitude control of signal energy (AM pulsing) and pulse width modulation control (PWM) and on/off keying (OOK). Modulation schemes include pulse modulation rates (1-10kHz) or frequency modulation rate (1-100 kHz). The radiation generated by the microwave generator 22 can be controlled by providing control signals to the microwave generator 22 thereby to select or vary one or more parameters of the modulation scheme.

In some embodiments, the generated radiation is modulated by control of a linear gain thereby to control power via a variable amplitude.

The microwave generator 22 is configured to delivery microwave radiation comprising a frequency between 900 MHz and 30 GHz, optionally wherein the frequency is about 915 MHz, about 2.45 GHz, about 5.8 GHz, about 8.0 GHz, or about 24.125 GHz.

In use, microwave radiation is generated by the microwave generator 22 in accordance with the operational parameters of the microwave generator 22 and supplied to the microwave applicator 26 via the cable 24. The applicator 26 is then provided at a surface of a biological tissue 28 and microwave radiation is delivered to the surface by the applicator 26.

The applicator 26 is then moved across the surface by the user. In the present embodiment, the applicator 26 is a hand-held applicator and is moved across the surface by hand. In the present embodiment, the sensor unit 30 senses position, velocity, acceleration, and orientation of the applicator 26 as it is moved across the surface. The controller 32 monitors the sensor output of the sensor unit 30 and provides control signals to the microwave generator 22 based on the monitored sensor output. In the present embodiment, as the applicator 26 is moved across the surface, the controller 32 monitors the sensor output to determine the position, velocity, acceleration and orientation of the applicator 26. One or more of the position, velocity, acceleration and orientation may be measured relative to a reference frame. Position may be determined relative to a reference point and may be referred to as displacement.

Based on the position, velocity, acceleration and orientation of the applicator 26, the controller 32 provides control signals to the microwave generator 22 to change or maintain one or more operational parameters of the microwave generator. In the present embodiment, the operational parameters are maintained and/or changed to maintain, increase or decrease the power of the generated microwave radiation by the generator 22. By maintaining, increasing or decreasing the power of the generated microwave radiation the microwave radiation is delivered to the surface in accordance with a pre-determined dose.

In the present embodiment, the control system unit 32 also modifies one or more operational parameters of the generator to change the AM or PWM based on the acceleration, orientation, speed, velocity and position input of the biological tissue 28 being treated as determined using sensor output from the further sensor unit 31.

The pre-determined energy dose is selected before the treatment starts. The selection is performed based on a number of factors, for example, the tissue type and/or condition to be treated. The energy dose may be, for example, an energy distribution that provides a controlled or constrained radiation delivery. For example, the energy distribution may be such that a uniform, non-uniform, optimum, or pre-set microwave energy density is delivered to the surface. In some embodiments, the energy dose is selected so that the heat treatment provided to the surface has a constant temperature or a temperature within a temperature window. In some embodiments, the energy dose is such that microwave radiation is provided inside a treatment area, for example, inside a pre-determined boundary.

For some treatment areas, different areas require different amounts of microwave radiation for treatment. Some conditions may require a more superficial treatment which would involve providing less power and a faster movement of the probe. Other conditions may require deeper heating and therefore slower movement of the probe to invoke radiating and conductive heating.

In some embodiments, it may be that the condition required different grades/levels of microwave treatment, and therefore more than one treatment region requiring different amounts of energy is provided. For example, the treatment area may have a sensitive area or a zone that is omitted (for example, a mole). As a further example, healthy tissue surface may be surrounded by a lesion to be treated.

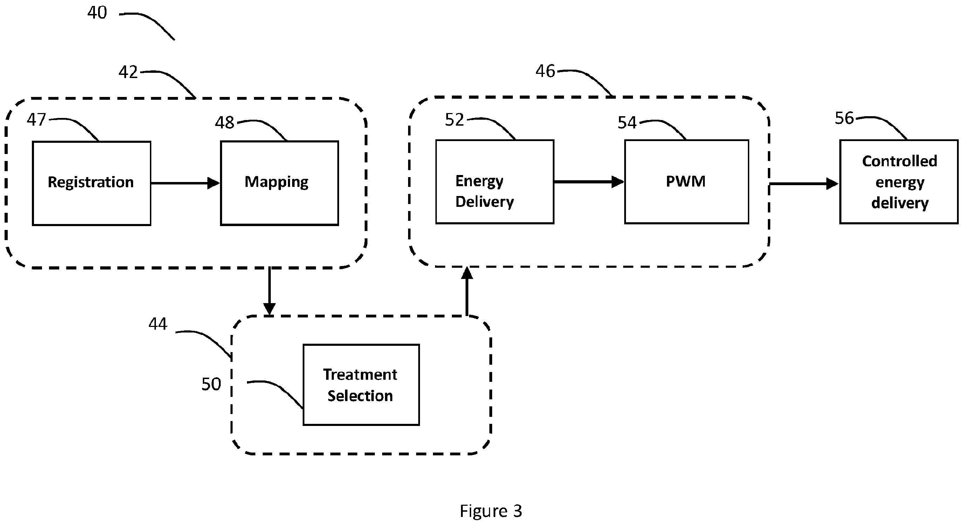

Figure 3 represents key phases of a process 40, in accordance with embodiments, for delivering and controlling an energy density over the surface of the biological tissue being treated. The process flow has a localising stage 42, in which a two dimensional map or 3D volume map of the treatment area is determined. 2D treatment areas and 3D treatment volumes may be referred to as a treatment region. The next stage 44 is an initialisation stage, in which one or more configuration parameters are selected. The next stage is the treatment stage 46.

Localising stage 42 comprises of spatial registration 47 of microwave applicator 26 and biological tissue 28. The spatial registration may occur in 2D or 3D space. This is followed by a mapping process 48 of the surface contour of the biological tissue 28 to acquire the boundary and surface geometry of the treatment area (the lesion) in 2D or 3D space. During the localising stage 42 no microwave power is supplied from the generator 22. The mapping process 48 may be performed by mapping circuitry associated with the controller 32 that receives sensor output and is configured to produce a map of the treatment area. In some embodiments, the 3D contour mapping could also be performed using, for example, LIDAR (Light Detection and Ranging) or ToF (Time-of-Flight) cameras.

As part of the system initialisation stage 44, one or more initialisation parameters are selected by a user. In particular, at treatment selection stage 50, parameters related to the desired energy dose or parameters related to a specific treatment based on a particular condition are selected. For example, in the present embodiments, a desired energy density is selected by a user based on the type of treatment required.

As part of the treatment stage 46, treatment is initiated at energy delivery step 52. At the energy delivery step 52, microwave power is generated from generator 22 through the microwave applicator assembly 26 on the biological tissue 28. The treatment is then performed substantially as described with reference to Figure 2.

Further at step 54, in the present embodiments, the control system unit 32 modifies one or more operational parameters of the generator to change the amplitude modulation (AM) or pulse-width modulation (PWM) of the generated microwaves. Step 54 may be referred to as operational parameter modifying stage 54. The changes are based on the acceleration, orientation, speed, velocity and position input of microwave applicator assembly 26 from IMU sensors unit 30. In the present embodiment, the control system unit 32 also modifies one or more operational parameters of the generator to change the AM or PWM based on the acceleration, orientation, speed, velocity and position input of biological tissue 28 being treated as determined using sensor output from further sensor unit 31.

At step 56 (controlled delivery stage), the control system unit 32 continuously monitors and executes controlled delivery of microwave energy density (measured in J/mm²) over the entire surface area to be treated.

In the above described process, a localising stage 42 is described. However, it will be understood that, in some embodiments, a map is not used or a map is provided without performing a mapping process. In some embodiments, only some co-ordinates of the treatment area are used during the treatment process. For example, only extreme values of height, width and length may be used.

In some embodiments, the localising stage 42 includes receiving treatment region data from an external source, for example, MRI, ultrasound or CT scan data. Any other medical imaging modality may provide treatment region data. In these embodiments, the obtained dataset is registered with the treatment region using the applicator 26 using a marker or other geometrical or anatomical features.

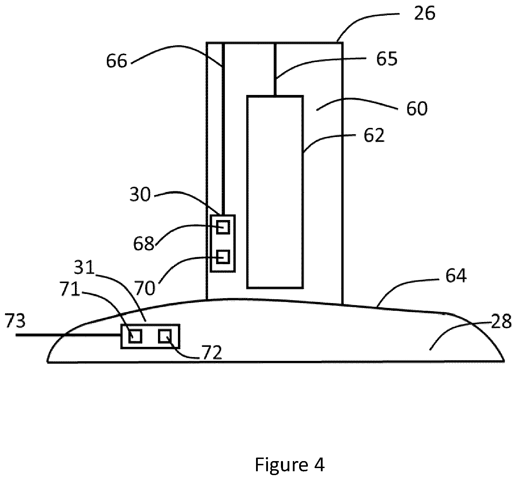

Figure 4 shows the microwave applicator assembly 26, in accordance with embodiments. The microwave applicator assembly 26 has applicator housing 60, microwave antenna 62 and sensor unit 30. The sensor unit 30 is as described with reference to Figure 2. The applicator 26 is shown as being applied on a nonhomogenous surface 64 of the biological tissue 28. The microwave applicator assembly 26 and sensor unit 30 are connected to the controller (not shown in Figure 4) by first cable 65 and second cable 66, respectively. While shown as first cable 65 and second cable 66, any wired or wireless connection suitable for carrying signals, for example, sensor data signals can be used in place of either first cable 65 or second cable 66.

In the present embodiment, the sensor unit 30 is an IMU sensor unit 30 and is equipped with a 3-axis accelerometer 68 and a gyroscope 70. These sensors (accelerometer 68 and gyroscope 70) can be used individually or in a combination with each other. In some embodiments, these sensors are combined in a single sensor and their location can be anywhere within the applicator housing 60. In use, the microwave antenna 62 may or may not be positioned in contact with the surface 64. Microwave antenna 62 is connected to the controller through the applicator housing 60 by means of the first cable 65. Sensor unit 30 is connected to the controller 32 through applicator housing 60 by the second cable 66.

Further sensor unit 31 is placed on the surface 64 of the tissue 28. The surface 64 corresponds to the surface of the body part being treated. Similarly to the sensor unit 30, the further sensor unit 31 consists of a 3-axis accelerometer 71 and a gyroscope 72 which can be used individually or in combination with each other. In some embodiments, the sensors of the further sensor unit 31 (accelerometer 71 and gyroscope 72) are combined in a single sensor and their location can be anywhere on the tissue 28. Further sensor unit 31 is wired to the controller 32 by means of third cable 73. In other embodiments, the further sensor unit 31 is connected to the controller 32 by a wireless connection and detects, for example, movements of the biological tissue.

In the above embodiment, a system for delivering radiation to a surface is described. In some embodiments, as described with reference to Figure 3, the system is configured to perform part of treatment area mapping process to determine the 2D or 3D position of the applicator and a surface contour of the tissue to be treated. The system is configured to determine a 2D or 3D map of the applicator displacement. The system is further configured to provide updates to the 2D or 3D map during treatment, for example, in real-time.

As an overview, initially a local 2D or 3D position of the applicator is registered spatially with a global co-ordinate system of the algorithm with the aid of IMU sensors such as accelerometer and gyroscope prior to the treatment. A boundary is then mapped manually using the applicator in "map mode". In some embodiments, the boundary is mapped by a user moving the applicator along the contour edge of the affected area on the surface of the tissue. In the "map mode" no microwave radiation is produced and data relevant for generating a map is collected. The applicator is moved along the said boundary in the 2D or 3D space generating 2D or 3D co-ordinate data representative of the surface of the tissue to be treated in the global co-ordinate system. The dataset can also include 4D generated time points with respect to each 3D co-ordinate being measured. The boundary coordinates of the area to be treated (the treatment area) are determined and hence provided in the control system.

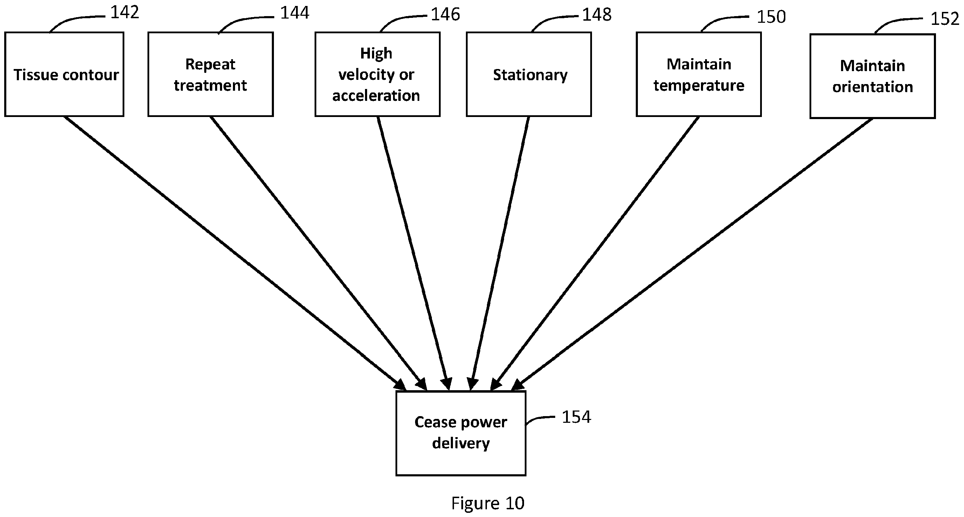

While treating the tissue such that when the applicator 26 is moved beyond the extent of the coordinates of the lesion, the controller 32 ceases the power and thus radiation delivery. This may provide an automatic safety feature, further described with reference to Figure 10, that prevents radiation delivery when during the treatment, applicator is not on target tissue boundary or surface, for example when the applicator leaves the surface accidently during the treatment or the orientation deviates from the "normal" surface vector.

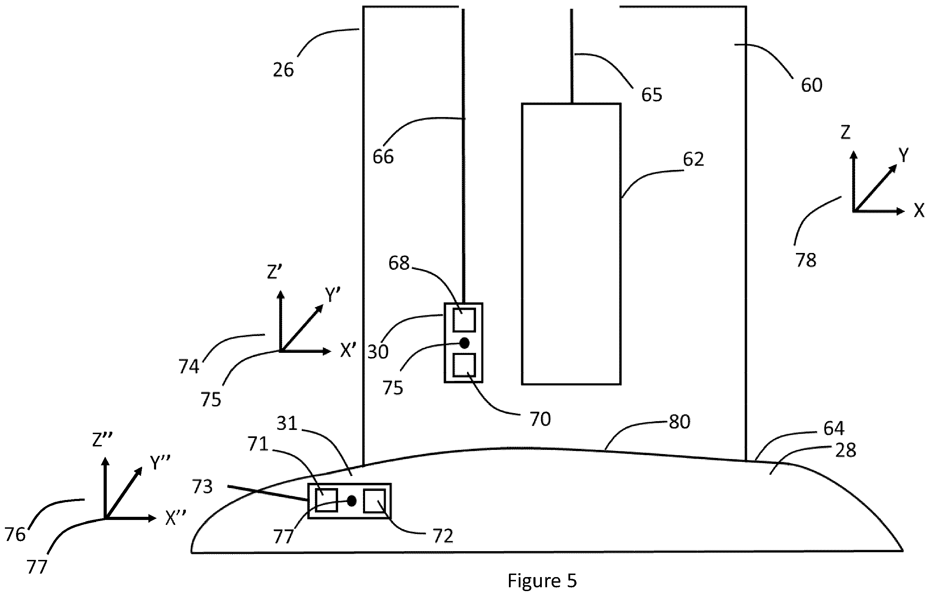

Figure 5 shows a cross-sectional view of the microwave applicator assembly 26 as described with reference to Figure 4. Figure 5 shows a first local co-ordinate system 74 having an origin 75 and three spatial axes (represented by X', Y' and Z'). The origin 75 of the local co-ordinate system 74 of the microwave applicator assembly 26 and thus of the microwave antenna 62 could be located anywhere on microwave applicator assembly 26. In the present embodiment, the origin 76 is located at the centre of the IMU sensor unit 30.

Similarly, a second local co-ordinate system 76 having an origin 77 and three spatial axes (represented by X", Y" and Z") represents spatial position of the further sensor unit 31. The origin 77 of the second local co-ordinate system 76 of the biological tissue 28 being treated could be located anywhere on the biological tissue 28 but preferably on the tissue surface 64. In the present embodiment, the origin 77 is located at the centre of the further sensor unit 31. In the present embodiment, the co-ordinate systems 74 and 76 are Cartesian co-ordinate systems, but it will be understood that, in other embodiments, any suitable co-ordinate system may be used.

To enable real-time and continuous tracking of the microwave applicator assembly 26 in 3D space, the first local co-ordinate system 74 with axes X', Y', Z' is registered with a global co-ordinate system 78 with axes X, Y and Z. Further, to enable real-time and continuous tracking of the biological tissue 28 being treated in 3D space, local coordinate system 63 with axes X", Y", Z" is registered with the global co-ordinate system 78 with axes X, Y and Z.

As a final step of the registration, the applicator 26 is moved across various points on the surface 64 of the tissue 28 recording the relative position of the distal end 80 of the applicator 26 and surface 64 of the tissue 28 in 2D and 3D space. This enables registration of the co-ordinate system 76 of biological tissue with the co-ordinate system 74 of applicator assembly 26 which in turn allows real-time tracking of the applicator assembly 26 with respect to the biological tissue 28 being treated.

An algorithm is provided that includes developing a spatial relationship between the three co-ordinate systems (74, 76, 78). This helps generate a common reference frame and reference frame transform between the microwave applicator assembly 26, IMU sensors unit 30, biological tissue 28, further sensor unit 31 and the global co-ordinate system 78 of the algorithm. Using this, the precise location of the sensors of sensor unit 30 and further sensor unit 31 (accelerometer 68, gyroscope 70, accelerometer 71, gyroscope 72), microwave applicator assembly 26, microwave antenna 62, distal end 80 of the microwave applicator assembly 26 and surface 64 of the biological tissue 28 can be all tracked in a singular global co-ordinate system 78 along X,Y,Z axes In addition, 2D and 3D position of the applicator 26 and tissue 28 can be tracked relative to each other. This feature may also provide a system suitable for treatments where keeping the tissue stationary for longer duration may not be possible. The transform may make processing easier and allows a re-mapping to be computed should the tissue move.

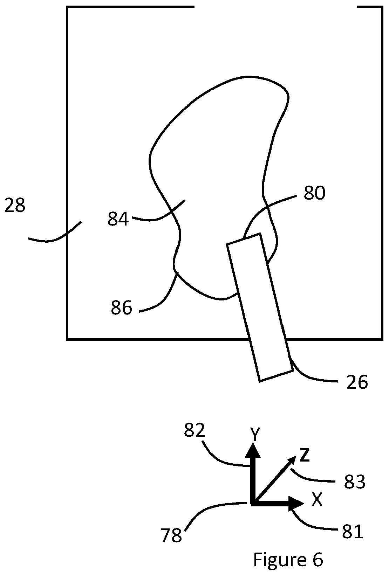

Figure 6 illustrates a 2D mapping process of a surface contour of the biological tissue 28. The map is generated along the X axis 81 and Y axis 82 of the global co-ordinate system 78 i.e. in the XY plane. The 3D data input from the sensors of sensor unit 30 and further sensor unit 31 (accelerometer 68, gyroscope 70, accelerometer 71, gyroscope 72) are processed to determine the position and orientation of the microwave applicator assembly 26 and biological tissue 28 in the 3D space.

The distal end 80 of the applicator assembly 26 may also be referred to as the treatment end of the applicator. In some embodiments, the distal end 80 has a diameter in the range of 1 to 30mm. In some embodiments, the distal end 80 has a diameter substantially equal to 6, 8, 10 or 12 mm. In some embodiments, the diameter of the distal end 80 may be selected depending on the dielectric properties and/or frequency of operation of the probe.

In the following, a mapping process for obtaining data representing a treatment area is described, in accordance with embodiments. Figure 6 shows a treatment area 84 (in this example, a lesion) of the biological tissue 28.

At first, the microwave applicator assembly 26 is moved smoothly, by an operator, along a surface contour of the biological tissue 28 to map an outline of the lesion. During the mapping, a boundary 86 of the treatment area 84 on the surface of the biological tissue 28 in the XY plane of the global co-ordinate system 78 is determined.

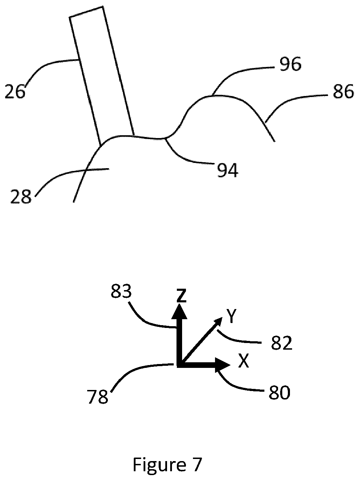

Figure 7 shows an alternative, cross sectional side view of the applicator assembly 26 applied to the surface 28. The microwave applicator assembly 26 is moved smoothly along the surface contour 86 of the biological tissue 28 to map the outline of the lesion as well as to locate and determine the extremes of the surface of the biological tissue 28 in the direction of the Z axis (the height) 83 of the global co-ordinate system 78 i.e. locating the minimum value of Z (Zmin), for example 94 and the maximum value of Z (Zmax), for example 96. The mapping process shown in the Figures 6 and 7 is described for illustrative purposes and in real-time the X, Y, Z co-ordinates will be preferably recorded simultaneously.

In the present embodiment, values of the Z co-ordinate are used as a safety feature. It will be understood that these values may also ensure that the entire 3D surface is covered.

Although described as part of a process for mapping the outline, in some embodiments, determining the maximum and minimum height of the treatment area in the Z direction may include performing a second mapping stage. For example, the maximum value of Z may not lie on the boundary of the treatment area and may lie inside the treatment area therefore a user must move the applicator to cover the treatment area before commencing the treatment.

In further embodiments, the system is configured to evaluate required energy settings i.e. the microwave power and time input based on the type of condition being treated. The applicator when moved in the map mode calculates surface or lesion contour and surface area (mm² or cm²). The user then selects the desired energy density J/mm² or J/cm² depending on various factors such as but not limited to tissue type, lesion type and surface area to be treated. Using the applicator and user input, the algorithm determines the required power (mW or W) and time required (s, min) and hence energy (J) required to treat the condition.

Depending upon the determined energy (J) and treatment area, the algorithm establishes velocity envelope for the applicator 26 movement throughout the treatment duration. This aspect of the algorithm can alert the user when the speed of the applicator is greater than the velocity envelope that is needed to deliver the determined or selected energy density.

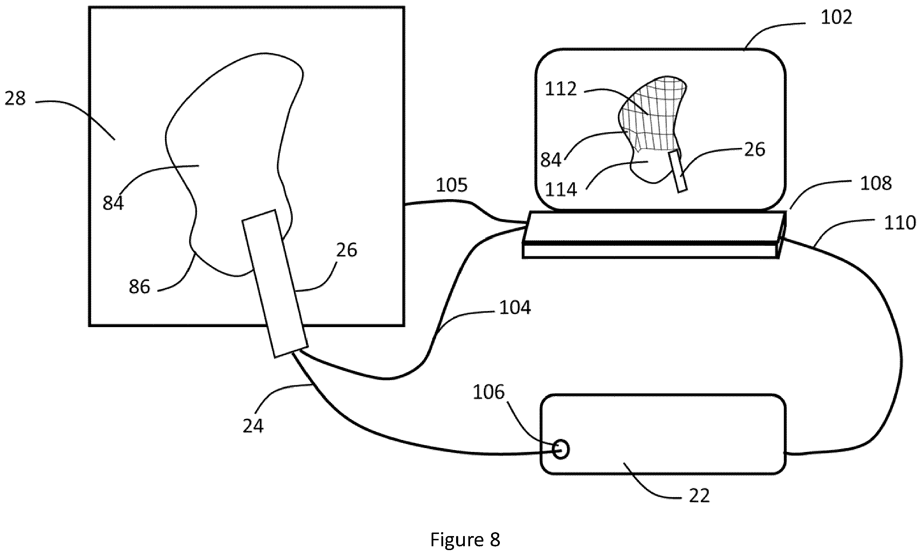

Figure 8 shows the microwave treatment system in accordance with embodiments. Figure 8 shows the system being used for treatment, with reference to the determined 2D/3D mapping of the biological tissue. Figure 8 shows the treatment in in real-time.

Figure 8 shows the microwave generator 22, the cable 24 and applicator assembly 26 as described with reference to Figure 2. Figure 8 also shows the biological tissue 28 that is being treated. Figure 8 shows a display 102 for displaying the determined 2D or 3D map to a user. As described with reference to Figure 2, the microwave applicator assembly 26 is connected to the microwave generator 22 via the cable assembly 24. The generator 22 has a port 106 to which the cable assembly 24 is connected.

A first wired connection 104 connects the sensor unit 30, which is provided as part of the microwave applicator assembly 26, to the controller 32. A second wired connection 105 connects the further sensor unit 31, which is provided at the biological tissue 28, to the controller 32. In the present embodiment, the controller 32 is provided as part of a computing resource 108, together with other circuity, for example, display circuitry and mapping circuitry. The controller 32 is provided as part of a processing circuitry or other suitable processing resource of the computing resource 108. The processing resource may also be referred to as an algorithm processing unit. Feedback data from the sensor unit 30 is communicated to the computing resource 108 via the first wired connection 104. Feedback data from the further sensor unit 31 is communicated to the computing resource 108 via the second wired connection 105. It will be understood that, in some embodiments, data from the sensor unit 30 and further sensor unit 31 may be communicated to the controller over a suitable wireless connection using wireless data transfer.

The computing resource 108 is connected to the microwave generator 22 via a further wired connection 110.

Although shown separately in Figure 8, it will be understood that in some embodiments, one or more of components may be integrated into a single system or piece of hardware with internal connections. For example, the display 102 and computing resource 108 may be provided as a single system. In a further example, the algorithm processing unit 108 may be an external computer or an internal unit that can perform required tasks. In further embodiments, in addition to the sensor unit, the controller is provided as part of the applicator. A single probe may be provided that includes integrated controller, CPU/firmware and sensors.

Turning to display 102, the display 102 and associated display circuitry of computer resource 108 are configured to display the determined map of the treatment area. The display circuitry is configured to update the display in real-time based on sensor output. In particular, the display is updated based on which parts of the treatment area have been treated.

In an embodiment, the display circuitry is configured to display the treatment area and graphical representation of where the applicator 26 has been moved i.e. the area that has been spanned by the movement of the applicator 26 during the treatment process. In the present embodiment, treated area 112 shown by a grid represents the displacement of the microwave applicator assembly 26 in the 2D/3D space hence providing 2D/3D footprints of the said applicator being moved continuously on the surface of the tissue being treated. The 2D/3D footprints of the applicator demonstrate the tissue surface that has already been treated. The display circuitry is also configured to display untreated area 114 representative of the area of the tissue that has not been treated.

The 2D or 3D displacement can be mapped in real-time by providing 2D or 3D footprints of the applicator 26 being moved continuously on the surface of the tissue being treated. As described above, the 2D or 3D footprints of the applicator can be used to differentiate between untreated tissue surface and the tissue surface that has already been treated. In some embodiments, the control system is configured to control the generator to cease or reduce the power delivery if the applicator is moved to an already treated tissue surface. For example, if the applicator 26 is moved to treated area 112, the power delivery is ceased or reduced. In some embodiments, when the applicator is moved to a previously treated area, the delivered power is determined based on the time elapsed since the applicator was previously at that location. In some embodiments, the 3D displacement is mapped in real-time providing 3D (Z) relating information that can be used to detect when the applicator is removed away from the tissue surface or is oriented in such a way that the applicator 26 is no longer normal to the surface 64 of biological tissue 28. As described in further detail with reference to Figure 10, this can form a safely mitigation and hence cease the delivery of power to the surface applicator.

The 2D/3D footprints of the applicator allow a user to see the size of treatment end of the applicator 26 in relation to the treatment area 84. For example, the applicator may provide a small focus represented by a fine point, or, for example, a 2cm diameter zone that can overlap by a particular fraction, for example, by 1/3.

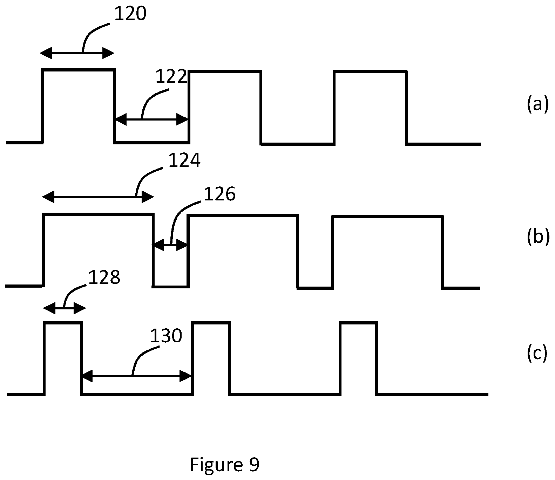

As described above, the power of generated microwave radiation and hence the energy density applied to a surface can be controlled by modulation. Figure 9 illustrates pulse width modulation sequences regulated by the controller 32 for delivering a controlled microwave energy density.

As an illustrative example, Figure 9 shows the case where a tissue treatment requires application of microwave radiation having a uniform energy density over the entire surface and treatment area of the lesion.

The control system is built in a closed-loop configuration where the control system modulates amplitude control of signal energy (AM pulsing) and/or PWM (pulse width modulation) duty cycle proportionally according the speed, velocity, orientation, displacement or acceleration of the microwave applicator delivering the energy thus keeping the delivered radiation density in the treated region uniform.

For example, as described in the following, a pre-determined velocity of 5 millimetres per second (referred to as Vbaseline) is assigned to the standard 50% duty cycle. Selection of Vbaseline can be performed automatically by the algorithm based on the condition and area of the lesion being treated. Alternatively, the user can provide feedback to provide an accepted velocity envelope.

Based on the preselected value, increase in the Vbaseline elicits an increase in the PWM duty cycle (by making Tₒₙ greater T_{off}) which in turn increases the intensity or magnitude of the power delivered at the given instance of time. Alternatively and/or additionally, decrease in the Vbaseline of the applicator treating the area produces decrease of the PWM duty cycle (i.e. Tₒₙ less than T_{off}) resulting in the decreased power intensity over the region being treated. This ensures that, for example, the radiation being delivered is uniform over the treated surface across the whole region. A continuous dose of radiation is thus modulated using PWM duty cycle where the 50% duty cycle relating to Vbaseline delivers power Pin. Pin refers to the actual power input specified that can be 0.1W to 100W but preferentially between 0.1W to 20W.

As described with reference to Figure 10, the control system can cease the power and hence the delivery if the velocity and acceleration of the applicator movement is outside a maximum specified value indicating the applicator is either being moved too fast or has been stationary for too long.

Figure 9(a), (b) and (c) show three pulse width modulation patterns for three different conditions.

The first pattern, of Figure 9(a) represents a 50% duty cycle where the period in which the signal is on (T_{ON}) is equal to the period in which the signal is off (T_{OFF}). T_{ON} is represented by numeral 120 and T_{OFF} is represented by numeral 122. In the present embodiment, the first example represents a modulation pattern for a condition when sensor output indicates that the velocity (V) of the microwave applicator assembly 26 is equal to a baseline value (Vbaseline). The baseline value is a pre-determined value. As an illustrative example, Vbaseline could be, for example, 5mm/sec or 10mm/sec etc. The value could be selected by a user or determined by an algorithm based on the condition and type of treatment required (i.e. the condition and type of treatment required could be selected by a user and the value is determined by the processing resource). Vbaseline dictates the control system unit 32 to maintain the 50% duty cycle of the PWM.

If the applicator 26 is moved such that the monitored sensor output indicates a velocity, V, of the microwave applicator assembly 26 to be less than Vbaseline (for example, in the event that the. operator slows down movement of the microwave applicator assembly 26 on the surface 28 of the biological tissue) then the controller provides control signals to the microwave generator to increase the PWM duty cycle. An example increased duty cycle is shown in Figure 9(b). T_{ON} (124) is greater than T_{OFF} (126). The controller 32, thus maintains the energy of the radiation being delivered to the surface to be substantially uniform in response to an increased velocity.

Furthermore, if the monitored sensor output indicates that the velocity, V of the microwave applicator assembly 26 has increased above Vbaseline (for example, if the microwave applicator assembly 26 is moved quicker than the Vbaseline across the surface 28 of the biological tissue) then the PWM duty cycle is decreased.

An example of a decreased duty cycle is shown in Figure 9(c). T_{ON} (128) is less than T_{OFF} (130). The controller 32 thus maintains the energy of the radiation being delivered to the surface to be substantially uniform in response to a decreased velocity.

In some embodiments, the change in duty cycle or other modulation parameter is determined in proportion to the change in one or more of speed, orientation, velocity, position and acceleration.

Therefore, the present invention demonstrates uniform and optimum microwave radiation delivery on large surface area of biological tissue 28 by compensating for the non-uniform and changing movement and displacement of microwave applicator assembly 26.

In some embodiments, the radiation delivery is controlled for microwave treatment which requires maintaining a precise temperature or a temperature within a temperature window over the entire duration of treatment. When the continuously tracked applicator returns back to a certain location to repeat the energy dose within a treatment, its current and previous time-points are known. Using this data, the time, location and evaluated temperature can be calculated and the radiation delivery can be modulated, for example by AM or PWM control, to maintain the temperature below a pre-set threshold. The determination of required microwave power may be determined in accordance with a mathematical relationship, for example, a bio-heat equation.

As an illustrative example, if the applicator is returned too quickly to a location that it has been previously visited, then for example PWM duty cycle is decreased (as illustrated in Figure 9(c) to decrease the radiation delivered to that location.

When treating certain types of conditions, the treatment efficacy may be improved when the temperature of the tissue temperature is maintained to a certain value or range for a certain period of time. Similarly, the cumulative equivalent time (in minutes) at 43°C also called as CEM43 model (Sapareto and Dewey, 1984), shows various temperature-time effects and is known to be used by thermal therapies to deliver specific thermal dose and to avoid thermal damage. (Rhoon, 2013; Mitra and Miller, 2017).

In the above describes embodiments, providing radiation for example, uniformly to a treatment area is described. The controller may be configured to monitor sensor output to maintain a required temperature window based on the condition to be treated by modulating radiation being delivered on the lesions surface. The temperature window could be in a range from 40-60 °C. The temperature may be substantially equal to precise values such as 41, 43, 45, 47, 48 °C or a temperature window such as 40-45, 41-44, 43-48 and 47-50 °C.

The temperatures are maintained for a pre-determined period of time, as a non-limiting example, this is between 1-600 seconds, in particular 30s, 60s, 120s, 180s, 240s, 300s and 600s. The preselected Vbaseline based on the surface area of the lesion being treated and user feedback provides a known energy density being distributed on the tissue surface. Using fundamental properties such as blood perfusion rate of the tissue being treated and the Penne's Bioheat equation (Pennes HH (1948)), the rate at which temperature of the tissue surface is increased or decreased is predicted by the algorithm for the preselected energy settings.

In some embodiments, the controller is configured to provide modulation of the energy density based on the calculated temperature decay of the surface. For example, for a condition such psoriasis and type of the treatment such as high dose of hyperthermia, the temperature window is selected to be 43-48 °C for a time period of 120s. Using the applicators map mode, the surface area of the tissue to be treated is recorded. During the treatment, the spatially tracked applicator is moved continuously on the surface being treated in a translation motion recording the timepoint and location of each 2D or 3D co-ordinate on the tissue surface that has been supplied with radiation. When the applicator returns to the already treated location, then the difference between the current spatial time point and the previous time point of radiation delivery provides temperature decay at that point. The energy density to the new time point at that location is then adjusted by the said control system by modulating the amplitude control of signal energy (AM pulsing) and/or PWM (pulse width modulation) duty cycle.

In further embodiments, the system also includes a failsafe mode, in which the microwave radiation being generated by the microwave generator is substantially reduced or switched off in response to a number of events. Figure 10 shows six conditions 142, 144, 146, 148, 150 and 152 that correspond to six events, in response to which the controller controls the microwave generator to ceases power generation or turns the power to 0W (represented by step 154).

The first condition 142 corresponds to the event when the monitored sensor output indicates that the applicator has reached or gone outside the boundary of the treatment area.

The second condition 144 corresponds to the event when the monitored sensor output indicates that the applicator 26 is moved back to an already treated position on the tissue surface. The second condition may only be implemented for certain radiation profiles, for example, only when a uniform energy density is required.

The third condition 146 corresponds to the event when the monitored sensor output is representative of the applicator 26 moving at a high velocity or acceleration. This may be determined by comparing the sensor output value to a threshold value.

The fourth condition 148 corresponds to the event when the monitored sensor output is representative of the application being stationary or static. This may be determined if the sensor output indicates that the applicator 26 is in substantially the same location for a period of time greater than a pre-determined threshold.

The fifth condition 150 corresponds to the event when the monitored sensor output is representative of the application being returns too soon or too quickly to a tissue surface during a treatment that is required to maintain a specific temperature as part of the treatment.

The sixth condition 152 corresponds to the event when the monitored sensor output is representative of the applicator 26 being orientated at an angle that is outside an angular range defined about the normal to (an axis perpendicular to) the treatment surface. It may be desirable to use the applicator 26 at an angle that is normal to the treatment surface and the sixth condition 152 corresponds to when the applicator is no longer at the desired, normal. In other embodiments, a different desired angle of application may be preferred and a condition may be such that that the monitored sensor output is representative of the applicator 26 being no longer oriented at the desired angle of application.

In response to any of these events, the controller controls the microwave generator to cut-out and cease generation of microwave radiation to prevent delivery of further microwave radiation from the applicator 26.

In some embodiments, the system has an indicator. The indicator may be provided which indicate that one or more of the above events has occurred. The indicator may be a visual or audio indicator. In some embodiments, the indicator could be provided on the display or on the applicator. 26

In the above described embodiments, the energy applied using the system constitutes delivering specified microwave power over a specified time. The microwave power could range from 0.1W to 100W but preferentially between 1W to 20W. The treatment time may range from one thousandth of a second to 1s and 1s to 1800s but preferably between 1s-10s. Surface power density may range from 1mW/ mm² to 20W/mm². The surface energy density may be in a range from 1mJ/mm² to 200J/mm².

The radiation applied can be in the form of a continuous oscillating electromagnetic wave (CW) at a fixed frequency or modulated (variable frequency). The frequency could range from 1MHz to 300GHz but preferentially could be in the microwave range from 0.9GHz to 16GHz.

In the above describe embodiments an applicator with a radiating antenna surface of is described.

In the above described embodiments, the microwave applicator is moveable by a user. The applicator can be moved over the entire surface in any direction. In example embodiments, the applicator is sized and/or shaped to be moved across the surface by hand, for example, like a paintbrush on canvas or a pen on paper.

In the above described embodiments, the sensor unit 30 is an inertial measurement unit (IMU) sensor. The IMU includes, for example, an accelerometer to measure the acceleration (A) of the applicator. The accelerometer may also provide sensor output representative of the velocity and displacement of the applicator.

In the above described embodiment, sensor unit also has a gyroscope added to the distal end of the applicator. A combination of the IMU sensors of sensor unit 30 can provide 6 degrees of freedom (DOF) tracking i.e. X, Y, Z displacement as well as pitch, roll, yaw i.e. θ, ψ, φ respectively.

By sensing orientation, an angle of application may be determined. During use, the applicator may be touching the surface but only at a glancing angle thus imparting a fraction of the energy expected/designed at the normal of 90 degrees e.g. normal or perpendicular angle of application. By sensing the angle of application, the use of the applicator may be optimised to increase or decrease the fraction of energy provided from the applicator.

Industrial 3-axis accelerometer and gyroscopes can be brought separately and embedded in one unit. Alternatively, 6-axis accelerometer and gyroscope mounted on a same chip can serve as one unit, for example, the 6-axis family devices package by InvenSense or the iNemo series by STMicroelectronics. The said accelerometer is used to detect magnitude and direction of the proper acceleration of the applicator. The acceleration and velocity of the applicator radiating energy in the tissue is continuously monitored over the given distance by the control system.

In the above described embodiments, pulse width modulation is described to control the power of the generated microwave radiation. It will be understood that other modulation schemes can be used, for example, pulse regimes including amplitude control of signal energy (AM pulsing) and pulse width modulation control (PWM) and on/off keying (OOK). Modulation schemes include pulse modulation rates (1-10kHz) or frequency modulation rate (1-100 kHz).

A continuous dose may be a fixed level of radiation or a modulated level of radiation during a treatment session. This continuous radiation delivery could be pulsed modulated one or five or fifty times a second during the ongoing treatment session. Preferentially continuous radiation delivery could be pulsed five times per second (5Hz).

The system described above provides inertial tracking of the biological tissue and the applicator assembly using IMU sensors providing 6 DOFs. It will be understood that other types of sensor may be used in combination or in place of the IMU sensors.

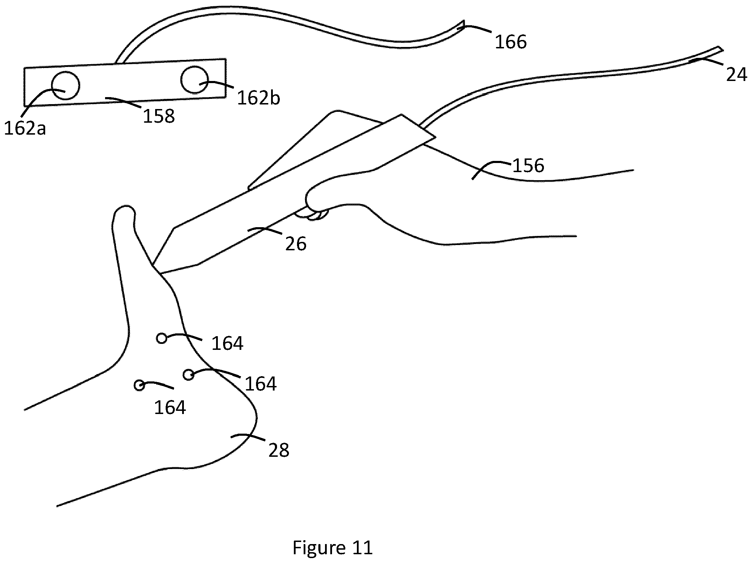

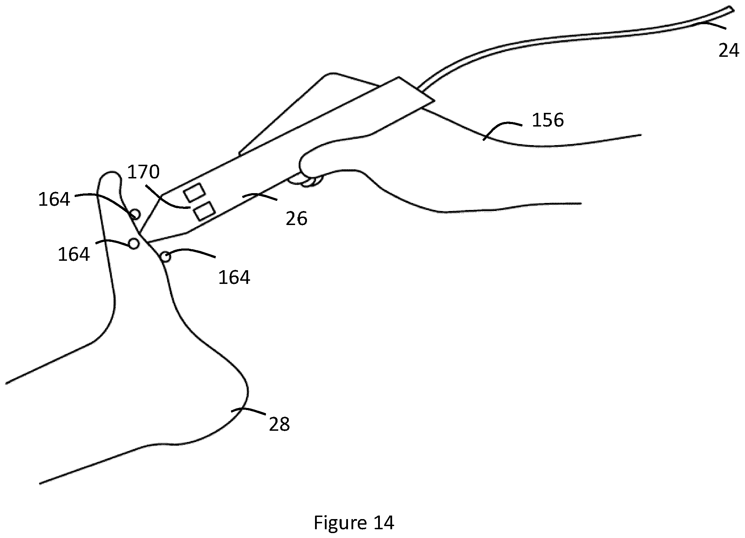

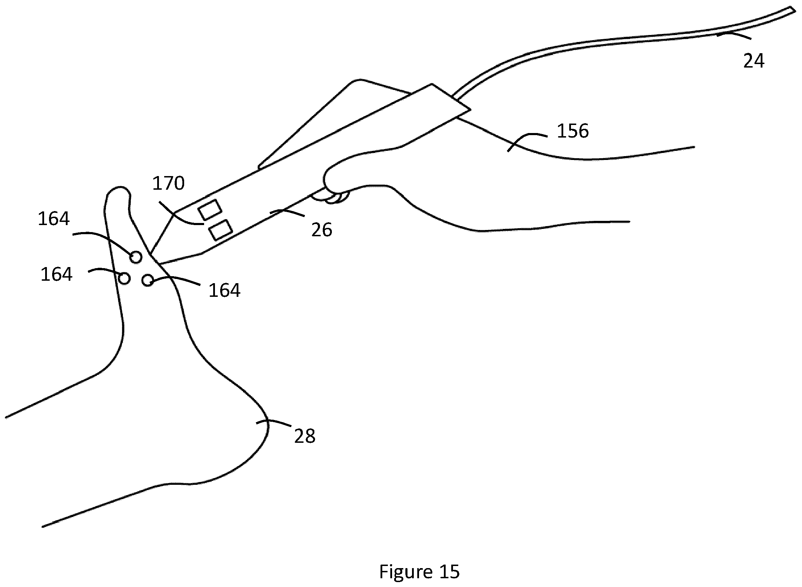

In a further embodiment, motion capture techniques such as optical tracking are employed. It is possible that sudden movement, for example, a sudden leg movement, could cause loss of registration, however by continuously tracking the biological tissue 28 and applicator assembly 26 using an optical tracking system this issue can be overcome thus allowing treatment of the correct areas. As described in further detail with reference to Figures 11 to 15, this alternate tracking is achieved by placing an active or passive set of markers on the biological tissue. One or more detecting cameras are then provided, externally or on the applicator assembly which would navigate the biological tissue and applicator assembly into a single co-ordinate system. In some embodiment, such a system is marker-less and can make use of anatomical features of the biological tissue being treated.

Figures 11 to 15 shows the system provided together with an optical tracking system, in accordance with further embodiments. Figure 11 shows part of microwave delivery system 20, as described with reference to Figure 2. Figure 11 shows applicator 26 and cable 24 of system 20. Applicator 26 has a sensor unit 30 (not shown in Figures 11 to 15) with IMU sensors. As shown in Figures 11 to 15, applicator 26 is moveable by a hand 156.

In Figure 11, a set of markers 164 is placed on the biological tissue 28. In Figure 11, the biological tissue 28 is part of a foot. The markers are tracked by an external camera system 158 comprising two cameras 162a, 162b. The camera system is connected to controller 32 via wired connection 166. In other embodiments, a wireless connection is provided.

In the embodiment of Figure 11, microwave applicator assembly 26 with IMU sensors is provided as described with reference to Figure 2. The camera 158 can visualise the set of markers 164 on the tissue 28 thus keeping the applicator assembly 26 and tissue 28 in a single co-ordinate system.

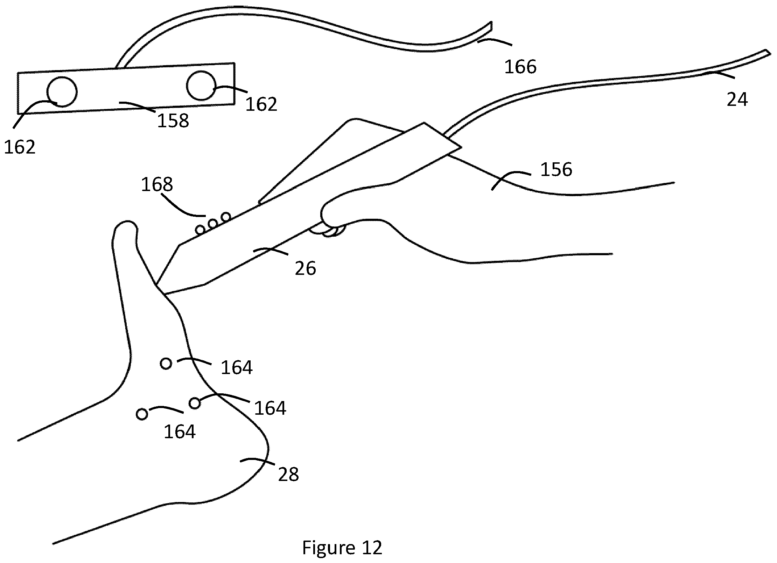

Figure 12 shows a further embodiment, in which a set of markers are provided on the applicator assembly 26 which is tracked by the external camera system 158, thus bringing tissue 28, applicator assembly 26 into a single co-ordinate system.

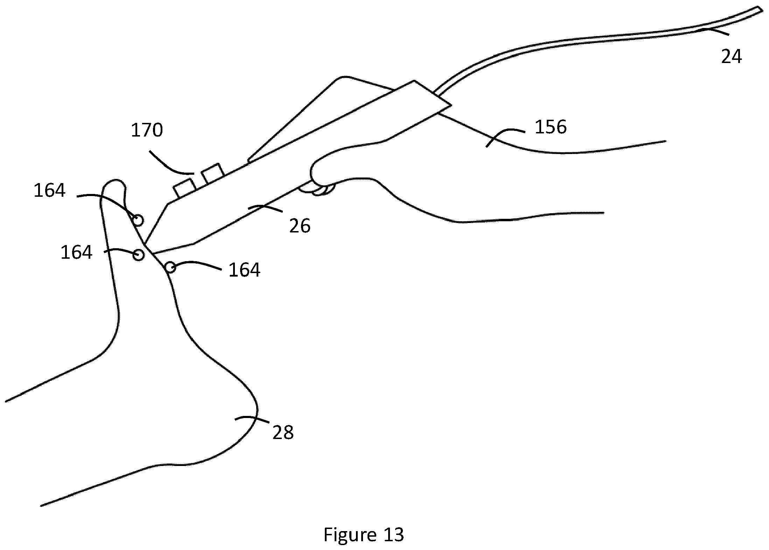

Figures 13, 14 and 15 show further embodiments which are substantially the same as the embodiment of Figure 12, except: with camera system 158 replaced by camera system 170 provided on applicator 26 (shown in Figure 13); alternative position of camera system 170 (Figure 13, 14 and 15) and with other possible configurations of markers 164 on tissue 28 (Figure 13, 14 and 15). In particular, Figure 13 shows markers provided on the tissue surface 28.

As illustrated in Figure 13, the camera system 170 can be also be implemented externally on the body of applicator assembly 26. Although shown as having two cameras, it may be that camera system (158 or 170) can have one, two or more cameras and can be placed anywhere on the applicator body. The camera system can visualise the set of markers 164 on the tissue 28 thus keeping the applicator assembly 26 and tissue 28 in single co-ordinate system.

The markers could be of any shape but preferably spherical. The markers may be active or passive. A passive marker can be covered with any material preferably any radiopaque material. An active marker comprises of an active source such as IRED (infrared emitting diodes) or LED (Light emitting diodes depending upon the detector camera type. Markers can be placed on prominent features of the tissue for example on the foot, on medial, lateral, dorsal or plantar side using bony palpations such as metarsal heads, metarsalphalangeal joint, cuboid etc or soft tissue palpations such as tibialis posterior tendon, or features such as transverse arch.

Although described as a camera detecting the position of the markers, in some embodiments, alternative marker detecting means for detecting the presence and/or position of one or more active or passive markers are provided. The alternative marker detecting means can be, for example, an electromagnetic system, a time of flight based system, a Lidar based system and/or a Doppler radar technique system. In some embodiments, the marker detecting means may use one or more sensors of the sensor 30 and/or further sensor 31.

The system described relates to treating larger surfaces of various tissues such as, but not limited to, skin, liver, spleen, kidney, lung, muscle, cardiac tissue, venous or arterial structures etc. In particular, the said method and apparatus relates to treating any surface of the biological tissue by means of supplying microwave radiation for ablative, non-ablative and coagulative purposes. The system can be used to treat a variety of conditions in tissues and in particular surface based treatments for example but not limited to in treating dermatological conditions.

The system can be used to treat biological conditions affecting mucosal linings for example and not limited to mucosal linings of oral, gastrointestinal, cervical tissues. Oral lesions for example and not limited to leukoplakia, hairy leukoplakia, lichen planus, xerostomia, mucositis, pyogenic granuloma, angioma, nicotinic stomatitis, actinic cheilitis, keratoacantoma, epithelial dysplasia, hyperkeratosis, oral candidosis, erythema migrans, canker sores.

The microwave treatment system is intended for example, for tissue hyperthermia, ablative, sub-ablative, non-ablative, coagulative and non-coagulative purposes. In addition, a method of continuously tracking the microwave radiation applicator in 2D or 3D space to control the radiation delivery is described. Further, another feature is an algorithm to maintain a desired temperature range of a tissue based on the condition and type of microwave treatment.

Though not exclusively, the present disclosure relates to treating conditions affecting epithelial tissues or conditions affecting said epithelial tissues such as cervical neoplasia, gastrointestinal neoplasia, gastric antral vascular ectasia, ulcerative colitis. In particular, the present invention relates to treating human dermatological conditions covering lesions with larger surface area than the applicator on the skin surface, for example and not limited to psoriasis vulgaris (plaque psoriasis), Inverse psoriasis, Pustular psoriasis, Erythrodermic psoriasis, Dermatitis, Seborrheic dermatitis, Eczema, Pruritus, certain fungal infection, thick and large Actinic Keratoses, cellulitis, melanoma and non-melanoma skin cancer such as Basal Cell Carcinoma and Squamous Cell Carcinoma etc.

The present invention also includes treating dermatological conditions in animals especially ones covering larger surface area than the applicator on the skin surface, for example and not limited to granulomatous and pyogranulomatous skin lesions, Papillomatosis (Fibropapillomas), Cellulitis/subcutaneous abscesses, Pruritus Dermatitis, Eczema, certain fungal infections, melanoma and non-melanoma skin cancer such as Basal Cell Carcinoma, Squamous Cell Carcinoma, Histiocytoma, Mast cell tumors, Plasmacytoma, Soft tissue sarcomas, Hair follicle tumors such as trichoepithelioma and pilomatricoma and other abnormal masses such as Calcinosus circumscripta.

In the above described embodiments, the controller controls the power of the generated microwave radiation. However, it will be understood that one or more other given characteristics of the microwave radiation that is generated may be controlled, in accordance with other embodiments. For example, the one or more characteristics include at least one of the frequency, frequency spectrum, power, power density, energy, energy density, intensity, strength, amount, magnitude, exposure time, dose, pulse duration, and pulse repetition rate of the microwave radiation.

A skilled person will appreciate that variations of the enclosed arrangement are possible without departing from the invention. Accordingly, the above description of the specific embodiment is made by way of example only and not for the purposes of limitations. It will be clear to the skilled person that minor modifications may be made without significant changes to the operation described.

## Claims

1. A microwave system, comprising:
a microwave generator (22);
a microwave applicator (26) for delivering microwave radiation generated by the microwave generator (22) to a surface, wherein the microwave applicator (26) is moveable relative to the surface;
one or more sensors (30) for sensing at least one of a position, an orientation, an acceleration, a speed and/or a velocity of the microwave applicator (26), and
a controller (32) configured to monitor sensor output from the one or more sensors (30) and further configured to control one or more operational parameters of the microwave system based at least on the monitored sensor output,
**characterized in that**
the controller is configured to control one or more operational parameters of the microwave generator (22) when the applicator (26) is at a position based on the time elapsed since the applicator (26) was previously at said position.

2. A microwave system as claimed in claim 1, wherein the controller is configured to control the one or more operational parameters thereby to maintain, increase and/or decrease a power or energy of the generated microwave radiation in response to the applicator (26) being moved relative to the surface, optionally, thereby to deliver microwave radiation to the surface in accordance with a pre-determined dose.

3. A microwave system according to any preceding claim, wherein the controller is configured to change the one or more operational parameters in response to a change in one or more of the position, orientation, speed, velocity, or acceleration of the applicator (26).

4. A microwave system according to any preceding claim, wherein the controller is configured to control the one or more operational parameters of the microwave generator (22) such that the microwave radiation delivered across the surface comprises a substantially constant energy density.

5. A microwave system according to any preceding claim, wherein the controller is configured to control the one or more operational parameters of the microwave generator (22) based on the sensor output so that the delivered microwave radiation maintains a temperature of the surface within a pre-determined temperature window.

6. A microwave system according to any preceding claim, wherein the controller is further configured to cut-off or at least reduce the amount of microwave radiation that is generated by the microwave generator (22) in response to at least one of:
a) the sensor output being representative of the microwave applicator (26) being outside a pre-determined treatment region;
b) the sensor output being representative of the microwave applicator (26) being at a height above a surface that is above a pre-determined threshold;
c) the sensor output being representative of the microwave applicator (26) being at a position or having an orientation, or moving at a speed or velocity or having an acceleration above an upper threshold and/or below a lower threshold;
d) the sensor output being representative of the microwave applicator (26) being oriented away from a desired axis relative to the surface

7. A microwave system as claimed in any preceding claim, wherein the generated microwave radiation comprises a series of pulsed microwave signals and the controller is configured to control the generation of microwave radiation by varying one or more pulse parameters.

8. A microwave system as claimed in any preceding claim, wherein the generated microwave radiation comprises modulated microwave signals and the controller is configured to control the generation of microwave radiation by varying one or more modulation parameters, optionally, wherein the modulation process comprises at least one of: amplitude modulation pulsing, pulse-width modulation and/or on/off keying.

9. A microwave system as claimed in any preceding claim, wherein the microwave applicator (26) is moveable by a user.

10. A microwave system as claimed in any preceding claim, wherein the controller is further configured to control the microwave generator (22) such that the applicator (26) delivers microwave radiation to a pre-determined treatment region of the tissue surface.

11. A microwave system as claimed in any preceding claim further comprising mapping circuitry configured to receive sensor output obtained during a mapping process and to determine said treatment region based at least on said sensor output.

12. A microwave system according to any preceding claim, wherein the microwave generator (22) is configured to deliver one of: microwave radiation at a fixed frequency, microwave radiation at a variable frequency and/or modulated microwave radiation.

13. A microwave system according to any preceding claim, wherein at least one of a), b), c). d) and e):
a) the microwave applicator (26) comprises a radiating antenna;
b) the controller and sensors (30) are provided as part of the applicator (26);
c) the system further comprises an indicator for providing an indication in response to at least one of the one or more sensor outputs being above and/or below a threshold;
d) the one or more sensor comprises: inertial measurement unit sensors, Microelectromechanical systems, accelerometer sensors, gyroscope sensors, an optical tracking system, one or more cameras, an electromagnetic tracking system, a time of flight system, a Lidar or Doppler radar system;
e) the microwave applicator (26) is coupled to the microwave generator (22) by a cable assembly.

14. A non-transitory computer readable medium comprising instructions operable by a processor to perform a method of controlling a microwave system, wherein the microwave system comprises a microwave applicator (26) configured to deliver microwave radiation generated by a microwave generator (22) to a surface and the microwave applicator (26) is further configured to be moved relative to the surface, wherein the method comprises:
monitoring sensor output from one or more sensors (30), wherein the sensor output from the one or more sensors (30) is representative of at least one of a position, an orientation, an acceleration, a speed and/or a velocity of a microwave applicator (26);
controlling one or more operational parameters of a microwave system based at least on the monitored sensor output;
wherein the controller is configured to control one or more operational parameters of the microwave generator (22) when the applicator (26) is at a position based on the time elapsed since the applicator (26) was previously at said position.

## Patentansprüche

1. Mikrowellensystem, das Folgendes umfasst:
einen Mikrowellengenerator (22),
einen Mikrowellenapplikator (26) zum Abgeben von Mikrowellenstrahlung, die durch den Mikrowellengenerator (22) erzeugt wird, an eine Oberfläche, wobei der Mikrowellenapplikator (26) im Verhältnis zu der Oberfläche beweglich ist,
einen oder mehrere Sensoren (30) zum Abfühlen mindestens eines von einer Position, einer Ausrichtung, einer Beschleunigung, einer Geschwindigkeit und/oder einer Schnelligkeit des Mikrowellenapplikators (26) und
eine Steuerung (32), die dafür konfiguriert ist, eine Sensorausgabe von dem einen oder den mehreren Sensoren (30) zu überwachen, und ferner dafür konfiguriert ist, einen oder mehrere Betriebsparameter des Mikrowellensystems auf Grundlage mindestens der überwachten Sensorausgabe zu steuern, und
**dadurch gekennzeichnet, dass**
die Steuerung dafür konfiguriert ist, einen oder mehrere Betriebsparameter des Mikrowellengenerators (22), wenn sich der Applikator (26) an einer Position befindet, auf Grundlage der Zeit, die vergangen ist, seit sich der Applikator (26) zuvor an der Position befand, zu steuern.

2. Mikrowellensystem nach Anspruch 1, wobei die Steuerung dafür konfiguriert ist, den einen oder die mehreren Betriebsparameter zu steuern, um dadurch eine Leistung oder Energie der erzeugten Mikrowellenstrahlung als Reaktion darauf aufrechtzuerhalten, zu steigern und/oder zu vermindern, dass der Applikator (26) im Verhältnis zu der Oberfläche bewegt wird, wahlweise, um dadurch Mikrowellenstrahlung entsprechend einer vorbestimmten Dosis an die Oberfläche abzugeben.

3. Mikrowellensystem nach einem der vorhergehenden Ansprüche, wobei die Steuerung dafür konfiguriert ist, den einen oder die mehreren Betriebsparameter als Reaktion auf eine Veränderung bei einem oder mehreren von der Position, Ausrichtung, Geschwindigkeit, Schnelligkeit oder Beschleunigung des Applikators (26) zu verändern.

4. Mikrowellensystem nach einem der vorhergehenden Ansprüche, wobei die Steuerung dafür konfiguriert ist, den einen oder die mehreren Betriebsparameter des Mikrowellengenerators (22) derart zu steuern, dass die Mikrowellenstrahlung, die über die Oberfläche abgegeben wird, eine im Wesentlichen konstante Energiedichte umfasst.

5. Mikrowellensystem nach einem der vorhergehenden Ansprüche, wobei die Steuerung dafür konfiguriert ist, den einen oder die mehreren Betriebsparameter des Mikrowellengenerators (22) auf Grundlage der Sensorausgabe derart zu steuern, dass die abgegebene Mikrowellenstrahlung eine Temperatur der Oberfläche innerhalb eines vorbestimmten Temperaturfensters erhält.

6. Mikrowellensystem nach einem der vorhergehenden Ansprüche, wobei die Steuerung ferner dafür konfiguriert ist, die Menge von Mikrowellenstrahlung, die durch den Mikrowellengenerator (22) erzeugt wird, als Reaktion auf mindestens eines von Folgendem abzuschalten oder mindestens zu verringern:
a) die Sensorausgabe ist repräsentativ dafür, dass sich der Mikrowellenapplikator (26) außerhalb eines vorbestimmten Behandlungsbereichs befindet,
b) die Sensorausgabe ist repräsentativ dafür, dass sich der Mikrowellenapplikator (26) bei einer Höhe oberhalb einer Oberfläche befindet, die oberhalb eines vorbestimmten Schwellenwertes liegt,
c) die Sensorausgabe ist repräsentativ dafür, dass sich der Mikrowellenapplikator (26) an einer Position befindet oder eine Ausrichtung aufweist oder sich mit einer Geschwindigkeit oder Schnelligkeit bewegt oder eine Beschleunigung aufweist, die oberhalb eines oberen Schwellenwertes und/oder unterhalb eines unteren Schwellenwertes liegen,
d) die Sensorausgabe ist repräsentativ dafür, dass der Mikrowellenapplikator (26) weg von einer gewünschten Achse im Verhältnis zu der Oberfläche ausgerichtet ist.

7. Mikrowellensystem nach einem der vorhergehenden Ansprüche, wobei die erzeugte Mikrowellenstrahlung eine Reihe von gepulsten Mikrowellensignalen umfasst und die Steuerung dafür konfiguriert ist, die Erzeugung von Mikrowellenstrahlung durch Variieren eines oder mehrerer Pulsparameter zu steuern.

8. Mikrowellensystem nach einem der vorhergehenden Ansprüche, wobei die erzeugte Mikrowellenstrahlung modulierte Mikrowellensignale umfasst und die Steuerung dafür konfiguriert ist, die Erzeugung von Mikrowellenstrahlung durch Variieren eines oder mehrerer Modulationsparameter zu steuern, wahlweise, wobei der Modulationsprozess mindestens eines von Folgendem umfasst: Amplitudenmodulationspulsung, Pulsweitenmodulation und/oder Ein-/Aus-Tastung.

9. Mikrowellensystem nach einem der vorhergehenden Ansprüche, wobei der Mikrowellenapplikator (26) durch einen Benutzer beweglich ist.

10. Mikrowellensystem nach einem der vorhergehenden Ansprüche, wobei die Steuerung ferner dafür konfiguriert ist, den Mikrowellengenerator (22) derart zu steuern, dass der Applikator (26) Mikrowellenstrahlung an einen vorbestimmten Behandlungsbereich der Gewebeoberfläche abgibt.

11. Mikrowellensystem nach einem der vorhergehenden Ansprüche, das ferner Abbildungsschaltungen umfasst, die dafür konfiguriert sind, eine Sensorausgabe zu empfangen, die während eines Abbildungsprozesses erhalten wurde, und den Behandlungsbereich auf Grundlage mindestens der Sensorausgabe zu bestimmen.

12. Mikrowellensystem nach einem der vorhergehenden Ansprüche, wobei der Mikrowellengenerator (22) dafür konfiguriert ist, eines von Folgendem abzugeben: Mikrowellenstrahlung mit einer festen Frequenz, Mikrowellenstrahlung mit einer veränderlichen Frequenz und/oder modulierte Mikrowellenstrahlung.

13. Mikrowellensystem nach einem der vorhergehenden Ansprüche, wobei mindestens eines von a), b), c), d) und e) gilt:
a) der Mikrowellenapplikator (26) umfasst eine abstrahlende Antenne,
b) die Steuerung und die Sensoren (30) werden als Teil des Applikators (26) bereitgestellt,
c) das System umfasst ferner eine Anzeigevorrichtung zum Bereitstellen einer Anzeige als Reaktion darauf, das mindestens eine von der einen oder den mehreren Sensorausgaben oberhalb und/oder unterhalb eines Schwellenwertes liegt,
d) der eine oder die mehreren Sensoren umfassen: Inertialmesseinheit-Sensoren, mikroelektromechanische Systeme, Beschleunigungsmesser-Sensoren, Kreiselsensoren, ein optisches Verfolgungssystem, eine oder mehrere Kameras, ein elektromechanisches Verfolgungssystem, ein Laufzeitsystem, ein Lidar- oder Doppler-Radarsystem,
e) der Mikrowellenapplikator (26) ist durch eine Kabelbaugruppe an den Mikrowellengenerator (22) gekoppelt.

14. Nicht-flüchtiges rechnerlesbares Medium, das Anweisungen umfasst, die durch einen Prozessor lauffähig sind, um ein Verfahren zum Steuern eines Mikrowellensystems durchzuführen, wobei das Mikrowellensystem einen Mikrowellenapplikator (26) umfasst, der dafür konfiguriert ist, Mikrowellenstrahlung, die durch einen Mikrowellengenerator (22) erzeugt wird, an eine Oberfläche abzugeben, und der Mikrowellenapplikator (26) ferner dafür konfiguriert ist, im Verhältnis zu der Oberfläche bewegt zu werden, wobei das Verfahren Folgendes umfasst:
Überwachen einer Sensorausgabe von einem oder mehreren Sensoren (30), wobei die Sensorausgabe von dem einen oder den mehreren Sensoren (30) repräsentativ für mindestens eines von einer Position, einer Ausrichtung, einer Beschleunigung, einer Geschwindigkeit und/oder einer Schnelligkeit eines Mikrowellenapplikators (26) ist,
Steuern eines oder mehrerer Betriebsparameter eines Mikrowellensystems auf Grundlage mindestens der überwachten Sensorausgabe,
wobei die Steuerung dafür konfiguriert ist, einen oder mehrere Betriebsparameter des Mikrowellengenerators (22), wenn sich der Applikator (26) an einer Position befindet, auf Grundlage der Zeit, seit sich der Applikator (26) zuvor an dieser Position befand, zu steuern.

## Revendications

1. Système à micro-ondes, comprenant :
un générateur de micro-ondes (22) ;
un applicateur de micro-ondes (26) pour délivrer un rayonnement micro-ondes généré par le générateur de micro-ondes (22) à une surface, dans lequel l'applicateur de micro-ondes (26) peut être déplacé par rapport à la surface ;
un ou plusieurs capteurs (30) pour détecter au moins une d'une position, d'une orientation, d'une accélération, d'une vitesse et/ou d'une vélocité de l'applicateur de micro-ondes (26) ; et
un dispositif de commande (32) configuré pour surveiller une sortie de capteur provenant des un ou plusieurs capteurs (30), et configuré en outre pour commander un ou plusieurs paramètres opérationnels du système à micro-ondes sur la base au moins de la sortie de capteur surveillée ;
**caractérisé en ce que** :
le dispositif de commande est configuré pour commander un ou plusieurs paramètres opérationnels du générateur de micro-ondes (22) lorsque l'applicateur (26) se trouve au niveau d'une position, sur la base du temps écoulé depuis que l'applicateur (26) se situait précédemment au niveau de ladite position.

2. Système à micro-ondes selon la revendication 1, dans lequel le dispositif de commande est configuré pour commander les un ou plusieurs paramètres opérationnels pour maintenir, accroître et/ou réduire une puissance ou une énergie du rayonnement micro-ondes généré en réponse au déplacement de l'applicateur (26) par rapport à la surface, pour délivrer ainsi optionnellement le rayonnement micro-ondes à la surface, conformément à une dose prédéterminée.

3. Système à micro-ondes selon l'une quelconque des revendications précédentes, dans lequel le dispositif de commande est configuré pour changer les un ou plusieurs paramètres opérationnels en réponse à un changement d'une ou de plusieurs de la position, de l'orientation, de la vitesse, de la vélocité ou de l'accélération de l'applicateur (26).

4. Système à micro-ondes selon l'une quelconque des revendications précédentes, dans lequel le dispositif de commande est configuré pour commander les un ou plusieurs paramètres opérationnels du générateur de micro-ondes (22) de sorte que le rayonnement micro-ondes délivré à travers la surface comprenne une densité d'énergie sensiblement constante.

5. Système à micro-ondes selon l'une quelconque des revendications précédentes, dans lequel le dispositif de commande est configuré pour commander les un ou plusieurs paramètres opérationnels du générateur de micro-ondes (22) sur la base de la sortie de capteur, de sorte que le rayonnement micro-ondes délivré maintienne une température de la surface dans le cadre d'une fenêtre de températures prédéterminée.

6. Système à micro-ondes selon l'une quelconque des revendications précédentes, dans lequel le dispositif de commande est en outre configuré pour couper ou au moins réduire la quantité de rayonnement micro-ondes qui est générée par le générateur de micro-ondes (22) en réponse à au moins un des faits suivants :
a) la sortie de capteur est représentative du fait que l'applicateur de micro-ondes (26) se situe à l'extérieur d'une région de traitement prédéterminée ;
b) la sortie de capteur est représentative du fait que l'applicateur de micro-ondes (26) se situe au niveau d'une hauteur au-dessus d'une surface qui se situe au-dessus d'un seuil prédéterminé ;
c) la sortie de capteur est représentative du fait que l'applicateur de micro-ondes (26) se situe au niveau d'une position, ou présente une orientation, ou se déplace à une vitesse ou à une vélocité, ou présente une accélération qui sont supérieures à un seuil supérieur et/ou inférieures à un seuil inférieur ;
d) la sortie de capteur est représentative du fait que l'applicateur de micro-ondes (26) est orienté à l'écart d'un axe voulu par apport à la surface.

7. Système à micro-ondes selon l'une quelconque des revendications précédentes, dans lequel le rayonnement micro-ondes généré comprend une série de signaux micro-ondes d'impulsion et le dispositif de commande est configuré pour commander la génération du rayonnement micro-ondes en faisant varier un ou plusieurs paramètres d'impulsion.

8. Système à micro-ondes selon l'une quelconque des revendications précédentes, dans lequel le rayonnement micro-ondes généré comprend des signaux micro-ondes modulés et le dispositif de commande est configuré pour commander la génération du rayonnement micro-ondes en faisant varier un ou plusieurs paramètres de modulation, dans lequel le processus de modulation comprend optionnellement au moins l'un des éléments suivants : une impulsion avec modulation en amplitude, une modulation d'impulsions en largeur et/ou une modulation tout ou rien.

9. Système à micro-ondes selon l'une quelconque des revendications précédentes, dans lequel l'applicateur de micro-ondes (26) peut être déplacé par un utilisateur.

10. Système à micro-ondes selon l'une quelconque des revendications précédentes, dans lequel le dispositif de commande est en outre configuré pour commander le générateur de micro-ondes (22) de sorte que l'applicateur (26) délivre le rayonnement micro-ondes à une région de traitement prédéterminée de la surface du tissu.

11. Système à micro-ondes selon l'une quelconque des revendications précédentes, comprenant en outre des circuits de cartographie configurés pour recevoir une sortie de capteur obtenue au cours d'un processus de cartographie et pour déterminer ladite région de traitement, au moins sur la base de ladite sortie de capteur.

12. Système à micro-ondes selon l'une quelconque des revendications précédentes, dans lequel le générateur de micro-ondes (22) est configuré pour délivrer l'un des rayonnements suivants : un rayonnement micro-ondes avec une fréquence fixe, un rayonnement micro-ondes avec une fréquence variable et/ou un rayonnement micro-ondes modulé.

13. Système à micro-ondes selon l'une quelconque des revendications précédentes, dans lequel au moins l'un de a), b), c), d) et e) s'applique :
a) l'applicateur de micro-ondes (26) comprend une antenne rayonnante ;
b) le dispositif de commande et les capteurs (30) sont fournis en tant que partie de l'applicateur (26) ;
c) le système comprend en outre un indicateur pour fournir une indication en réponse au fait qu'au moins une des une ou plusieurs sorties de capteur se situe au-dessus et/ou en-dessous d'un seuil ;
d) les un ou plusieurs capteurs comprennent : des capteurs d'unité de mesure inertielle, des systèmes micro-électromécaniques, des capteurs accélérométriques, des capteurs gyroscopiques, un système de suivi optique, une ou plusieurs caméras, un système de suivi électromagnétique, un système de temps de vol, un système de radar Lidar ou Doppler ;
e) l'applicateur de micro-ondes (26) est couplé au générateur de micro-ondes (22) par un ensemble de câbles.

14. Support lisible par ordinateur non transitoire comprenant des instructions pouvant être mises en oeuvre par un processeur pour exécuter un procédé de commande d'un système à micro-ondes, dans lequel le système à micro-ondes comprend un applicateur de micro-ondes (26) configuré pour délivrer un rayonnement micro-ondes, généré par un générateur de micro-ondes (22), à une surface, et l'applicateur de micro-ondes (26) est en outre configuré pour être déplacé par rapport à la surface, dans lequel le procédé comprend les étapes suivantes :
surveillance d'une sortie de capteur provenant d'un ou de plusieurs capteurs (30), dans lequel la sortie de capteur des un ou plusieurs capteurs (30) est représentative d'au moins une d'une position, d'une orientation, d'une accélération, d'une vitesse et/ou d'une vélocité d'un applicateur de micro-ondes (26) ;
commande d'un ou de plusieurs paramètres opérationnels d'un système à micro-ondes, sur la base d'au moins la sortie de capteur surveillée ;
dans lequel le dispositif de commande est configuré pour commander un ou plusieurs paramètres opérationnels du générateur de micro-ondes (22) lorsque l'applicateur (26) se trouve au niveau d'une position, sur la base du temps écoulé depuis que l'applicateur (26) se trouvait précédemment au niveau de ladite position.
